# EUROPEAN PATENT APPLICATION

(11) **EP 3 954 783 A1**
(43) Date of publication of application: **16.02.2022**
(21) Application number: 20190953.8
(22) Date of filing: 13.08.2020
(51) Int. Cl.: C12Q 1/6816, C12N 9/22

(54) **POLYNUCLEOTIDE DETECTION BY CAS NUCLEASES**

(71) Applicant: Deutsches Krebsforschungszentrum, 69120 Heidelberg (DE)
(72) Inventor: The designation of the inventor has not yet been filed
(74) Representative: Altmann Stößel Dick Patentanwälte PartG mbB

(57) **Abstract**

The present invention relates to a method for detecting a target RNA and/or a target DNA in a sample of a subject, the method comprising the steps of a) contacting said sample with a Type V Cas nuclease, a Type V crRNA targeting said target DNA, a type VI Cas nuclease, a Type VI crRNA targeting said target RNA, a reporter RNA and a reporter DNA; and b) detecting cleavage of said reporter RNA, thereby detecting said target RNA; and/or detecting cleavage of said reporter DNA, thereby detecting said target DNA, and to methods uses and means related thereto.

SEQUENCE LISTING

<110>  Deutsches Krebsforschungszentrum

<120>  Polynucleotide detection by Cas nucleases

<130>  DK16159AEP

<160>  25

<170>  PatentIn version 3.5

<210>  1
<211>  30
<212>  DNA
<213>  Artificial Sequence

<220>
<223>  S-RPA-Forward

<400>  1
tcctaggttg aagataaccc acataataag                                        30


<210>  2
<211>  30
<212>  DNA
<213>  Artificial Sequence

<220>
<223>  S-RPA-Forward

<400>  2
tcctaggttg aagataaccc acataataag                                        30


<210>  3
<211>  55
<212>  DNA
<213>  Artificial Sequence

<220>
<223>  Orf1ab-RPA-Forward

<400>  3
gaaattaata cgactcacta tagggcgaag ttgtaggaga cattatactt aaacc            55


<210>  4
<211>  31
<212>  DNA
<213>  Artificial Sequence

<220>
<223>  Orf1ab-RPA-Reverse

<400>  4
tagtaagact agaattgtct acataagcag c                                      31


<210>  5
<211>  64
<212>  RNA

<213>  Artificial Sequence

<220>
<223>  S-crRNA

<400>  5
gauuuagacu accccaaaaa cgaaggggac uaaaacgcag caccagcugu ccaaccugaa      60

gaag                                                                  64


<210>  6
<211>  64
<212>  RNA
<213>  Artificial Sequence

<220>
<223>  Orf1ab-crRNA

<400>  6
gauuuagacu accccaaaaa cgaaggggac uaaaacccaa ccucuucugu aauuuuuaaa      60

cuau                                                                  64


<210>  7
<211>  14
<212>  RNA
<213>  Artificial Sequence

<220>
<223>  lateral flow reporter RNA


<220>
<221>  misc_feature
<222>  (1)..(1)
<223>  2'-O-methyl-adenosine

<220>
<221>  misc_feature
<222>  (1)..(1)
<223>  6-carboxyfluorescein

<220>
<221>  misc_feature
<222>  (7)..(7)
<223>  2'-O-methyl-adenosine

<220>
<221>  misc_feature
<222>  (8)..(8)
<223>  2'-O-methyl-adenosine

<220>
<221>  misc_feature
<222>  (14)..(14)
<223>  biotin

<220>
<221>  misc_feature
<222>  (14)..(14)
<223>  2'-O-methyl-adenosine

<400>  7
aauggcaaau ggca                                                         14


<210>  8
<211>  56
<212>  DNA
<213>  Artificial Sequence

<220>
<223>  N-RPA-Forward_v1

<400>  8
gaaattaata cgactcacta tagggtaatc agacaaggaa ctgattacaa acattg           56


<210>  9
<211>  64
<212>  DNA
<213>  Artificial Sequence

<220>
<223>  N-RPA-Reverse_v1

<400>  9
gacttccatg ccaatgcgcg acattccgaa gagacttcca tgccaatgcg cgacattccg       60

aaga                                                                    64


<210>  10
<211>  56
<212>  DNA
<213>  Artificial Sequence

<220>
<223>  N-RPA-Forward_v2

<400>  10
gaaattaata cgactcacta tagggactaa tcagacaagg aactgattac aaacat           56


<210>  11
<211>  32
<212>  DNA
<213>  Artificial Sequence

<220>
<223>  N-RPA-Reverse_v2

<400>  11
cacgttcccg aaggtgtgac ttccatgcca at                                     32


<210>  12
<211>  58
<212>  DNA
<213>  Artificial Sequence

<220>
<223>  E-RPA-Forward_v1

<400>  12
gaaattaata cgactcacta taggggttaa tagttaatag cgtacttctt tttcttgc        58


<210>  13
<211>  31
<212>  DNA
<213>  Artificial Sequence

<220>
<223>  E-RPA-Reverse_v1

<400>  13
ttaacaatat tgcagcagta cgcacacaat c                                     31


<210>  14
<211>  58
<212>  DNA
<213>  Artificial Sequence

<220>
<223>  E-RPA-Forward_v2

<400>  14
gaaattaata cgactcacta tagggatagt taatagcgta cttctttttc ttgctttc        58


<210>  15
<211>  31
<212>  DNA
<213>  Artificial Sequence

<220>
<223>  E-RPA-Reverse_v2

<400>  15
tacaagactc acgttaacaa tattgcagca g                                     31


<210>  16
<211>  64
<212>  RNA
<213>  Artificial Sequence

<220>
<223>  N-crRNA

<400>  16
   gauuuagacu accccaaaaa cgaaggggac uaaaacgaac gcugaagcgc ugggggcaaa       60
   uugu                                                                   64


<210>  17
<211>  64
<212>  RNA
<213>  Artificial Sequence

<220>
<223>  E-crRNA

<400>  17

gauuuagacu accccaaaaa cgaaggggac uaaaacucga agcgcaguaa ggauggcuag 60

ugua 64

<210> 18
<211> 11
<212> DNA
<213> Artificial Sequence

<220>
<223> reporter DNA sequence

<400> 18
ttattattat t 11

<210> 19
<211> 11
<212> DNA
<213> Artificial Sequence

<220>
<223> fluorescence reporter DNA

<220>
<221> misc_feature
<222> (1)..(1)
<223> 6-carboxyfluorescein

<220>
<221> misc_feature
<222> (11)..(11)
<223> Iowa Black® FQ

<400> 19
ttattattat t 11

<210> 20
<211> 11
<212> DNA
<213> Artificial Sequence

<220>
<223> lateral flow reporter DNA -1

<220>
<221> misc_feature
<222> (1)..(1)
<223> 6-carboxyfluorescein

<220>
<221> misc_feature
<222> (11)..(11)
<223> biotin

<400> 20
ttattattat t 11

<210>  21
<211>  11
<212>  DNA
<213>  Artificial Sequence

<220>
<223>  lateral flow reporter DNA - 2


<220>
<221>  misc_feature
<222>  (1)..(1)
<223>  6-carboxyfluorescein

<220>
<221>  misc_feature
<222>  (11)..(11)
<223>  digoxigenin

<400>  21
ttattattat t                                                            11


<210>  22
<211>  11
<212>  DNA
<213>  Artificial Sequence

<220>
<223>  lateral flow reporter DNA - 3


<220>
<221>  misc_feature
<222>  (1)..(1)
<223>  digoxigenin

<220>
<221>  misc_feature
<222>  (11)..(11)
<223>  biotin

<400>  22
ttattattat t                                                            11


<210>  23
<211>  41
<212>  RNA
<213>  Artificial Sequence

<220>
<223>  Type V crRNA (Example 3)

<400>  23
uaauuucuac uaaguguaga ugaccugcga gcggguucug a                           41


<210>  24
<211>  6
<212>  RNA
<213>  Artificial Sequence

<220>
<223>  lateral flow reporter RNA-2


<220>
<221>  misc_feature
<222>  (1)..(1)
<223>  6-carboxyfluorescein

<220>
<221>  misc_feature
<222>  (6)..(6)
<223>  biotin

<400>  24
uuuuuu                                                                  6


<210>  25
<211>  6
<212>  DNA
<213>  Artificial Sequence

<220>
<223>  fluorescence reporter RNA


<220>
<221>  misc_feature
<222>  (1)..(1)
<223>  TEX615

<220>
<221>  misc_feature
<222>  (1)..(1)
<223>  deoxyribonucleotide

<220>
<221>  misc_feature
<222>  (1)..(1)
<223>  phosphorothioate bond to the following nucleotide

<220>
<221>  misc_feature
<222>  (2)..(2)
<223>  phosphorothioate bond to the following nucleotide

<220>
<221>  misc_feature
<222>  (2)..(2)
<223>  deoxyribonucleotide

<220>
<221>  misc_feature
<222>  (3)..(3)
<223>  ribonucleotide

<220>
<221>  misc_feature
<222>  (4)..(4)
<223>  ribonucleotide

<220>

<221>  misc_feature
<222>  (5)..(5)
<223>  deoxyribonucleotide

<220>
<221>  misc_feature
<222>  (6)..(6)
<223>  phosphorothioate bond to the following nucleotide

<220>
<221>  misc_feature
<222>  (6)..(6)
<223>  deoxyribonucleotide

<220>
<221>  misc_feature
<222>  (6)..(6)
<223>  Iowa Black® RQ-Sp

<400>  25
taaugc                                                                 6

## Description

The present invention relates to a method for detecting a target RNA and/or a target DNA in a sample of a subject, the method comprising the steps of a) contacting said sample with a Type V Cas nuclease, a Type V crRNA targeting said target DNA, a type VI Cas nuclease, a Type VI crRNA targeting said target RNA, a reporter RNA and a reporter DNA; and b) detecting cleavage of said reporter RNA, thereby detecting said target RNA; and/or detecting cleavage of said reporter DNA, thereby detecting said target DNA, and to methods uses and means related thereto.

The new SARS-like coronavirus SARS-CoV-2 (also known as 2019-nCoV), the virus causing coronavirus disease 2019 (COVID-19), is spreading fast throughout the worldwide population. Currently, there is no specific therapy or vaccination known. Prevention of transmission is based on isolation quarantine of carriers. Equally, potential carriers are usually placed under quarantine. However, a vast majority of infected people do not develop noticeable clinical symptoms. RT-PCR tests can identify subjects who are currently infected with SARS-CoV-2; however, such tests require specific equipment, which may not be available in less developed countries.

CRISPR (Clustered, Regularly Interspaced Short Palindromic Repeats) systems in bacteria and archaea mediate specific degradation of foreign, invading nucleic acids. They comprise a CRISPR-associated (Cas) nuclease which can be programmed by short guideRNAs (gRNAs) to induce double-strand breaks at specific, sequence-complementary DNA loci. Besides applications in modification of the genetic composition of cells, CRISPR system have also been used in molecular detection of specific sequences, single-nucleotide variants, and patogens (cf. e.g. Zhou et al. (2018), J Cell Mol Med 22:5807). Diverse CRISPR-Cas systems have been adopted for genome engineering in mammalian cells and animals, most prominently the CRISPR-Cas9 system from Streptococcus pyogenes (SpyCas9). CRISPR Cas type VI systems are unusual in that the nuclease is an RNase. Moreover, once activated by a ssRNA sequence complementary to their crRNA spacer, type VI Cas nucleases have unspecific RNase activity. This property was used by Kellner et al. (2019), Nature Protocols 14:2986 to establish an assay system testing for the presence of specific polynucleotide with fluorescence and lateral flow readouts. The method was later applied to detection of Coronavirus SARS-CoV-2 (Zhang et al. (2020), "SHERLOCK COVID-19 Testing Kit Instructions", v. 20200321, (www.broadinstitute.org/files/publications/special/COVID-19 detection (updated).pdf). A similar assay using a Cas12 nuclease, which has DNase activity, was provided recently by Joung et al. (2020), "Point-of-care testing for COVID-19 using SHERLOCK diagnostics", v. 20200505 (www.stopcovid.science/docs/STOPCovid Whitepaper.pdf and Huang et al. (2020), Biosensors and Bioelectronics 164:112316). Nonetheless, also these methods require incubations at specific temperatures, which may not be possible to achieve where appropriate equipment is not available. Recently, saliva was proposed as a suitable sample material for SARS-CoV-2 detection (Wyllie et al. (2020), medRxiv 2020.04.16.20067835; doi.org/10.1101/2020.04.16.20067835).

There is, thus, a need in the art for improved means and methods for detecting RNA virus infection, in particular SARS-CoV-2 infection, avoiding the drawbacks ad requirements of the prior art. This problem is solved by the means and methods disclosed herein with the features of the independent claims. Advantageous embodiments which might be realized in an isolated fashion or in any arbitrary combinations are listed in the dependent claims.

In accordance, the present invention relates to a method for detecting an RNA virus in a sample, comprising the steps of
a) releasing viral RNA from said sample;
b) amplifying at least parts of the viral RNA comprised in said sample;
c) contacting the amplified viral RNA of step b) with a Type VI Cas nuclease, a crRNA, and a reporter RNA; and
d) detecting cleavage of said reporter RNA, thereby detecting said RNA virus.

The method of the present invention is an in vitro method. Moreover, it may comprise steps in addition to those explicitly mentioned above. For example, further steps may relate, e.g., to providing a sample for step a), or contacting the viral RNA with further reagents in steps b), c) and/or d). Moreover, one or more of said steps may be aided or performed by automated equipment, however, preferably specialized equipment is not necessary to perform the method in its entirety.

In general, terms used herein are to be given their ordinary and customary meaning to a person of ordinary skill in the art and, unless indicated otherwise, are not to be limited to a special or customized meaning. As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. Also, as is understood by the skilled person, the expressions "comprising a" and "comprising an" preferably refer to "comprising one or more", i.e. are equivalent to "comprising at least one".

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also, preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1% by weight, most preferably less than 0.1% by weight of non-specified component(s).

The term "fragment" of a biological macromolecule, preferably of a polynucleotide or polypeptide, is used herein in a wide sense relating to any sub-part, preferably subdomain, of the respective biological macromolecule comprising the indicated sequence, structure and/or function. Thus, the term includes sub-parts generated by actual fragmentation of a biological macromolecule, but also sub-parts derived from the respective biological macromolecule in an abstract manner, e.g. in silico.

The term "RNA virus" is known to the skilled person to relate to any virus having RNA as a genome. Preferably, the virus is a human and/or animal pathogenic virus, more preferably a human pathogenic virus. Preferably, the RNA virus is a virus having single-stranded RNA as a genome, preferably is a coronavirus or an influenza virus. More preferably, the RNA virus is a coronavirus as specified herein below. In accordance with the above, "viral RNA" preferably is the RNA a viral particle carries as a genome, or a fragment thereof. However, viral RNA preferably may also comprise free, i.e. non-capsid bound, viral RNA produced by an infected cell.

The term "coronavirus" is understood by the skilled person to relate to a group of viruses from the order Nidovirales, having a positive-sense single-stranded RNA genome with a size of approx. 25 to 35 kilobases. Preferably, the coronavirus is a beta-coronavirus, more preferably a severe acute respiratory syndrome coronavirus (SARS-CoV)-2, SARS-CoV-1, or Middle East respiratory syndrome coronavirus (MERS-CoV). Preferably the coronavirus is SARS-CoV-2. Preferably, the terms "SARS-CoV-2" and "severe acute respiratory syndrome coronavirus 2" relate to the virus identified in Genbank entry NCBI:txid2697049. Symptoms and diseases caused by coronavirus infection and in particular SARS-CoV-2 infection are known to the skilled person. In a preferred embodiment, the coronavirus is HCoV-NL63, HCoV-229E, HCoV-OC43, and HCoV-HKU1, all of which are known to the skilled person.

Thus, in a preferred embodiment, the RNA virus is selected from the list consisting of SARS-CoV-2, an influenza virus, HCoV-NL63, HCoV-229E, HCoV-OC43, and HCoV-HKU1. In case two RNA viruses shall be detected, they are, in a preferred embodiment, SARS-CoV-2 and an influenza virus.
The term "sample" refers to a sample of biological origin, preferably a sample from a subject. Preferably, the sample is a sample of separated cells or a sample from a tissue or an organ. Tissue or organ samples may be obtained from any tissue or organ by, e.g., biopsy. Separated cells may be obtained from the body fluids, such as lymph, blood, plasma, serum, liquor and other, or from the tissues or organs by separating techniques such as centrifugation or cell sorting. Preferably, the sample is a tissue or body fluid sample which is known or suspected to comprise an RNA virus. More preferably the sample is a sample of a body fluid, preferably saliva, sputum, blood, plasma, serum, lacrimal fluid, nasal discharge, urine, or stool sample. Preferred samples are from saliva, sputum, nasal discharge, and nasal swabs; most preferably, the sample is a saliva or sputum sample. The sample can be obtained from the subject by routine techniques which are well known to the person skilled in the art, e.g., venous or arterial puncture, nasal swabs, or open biopsy including aspiration of tissue or cellular material from a subj ect.

The term "releasing" is, in principle, understood by the skilled person. As used herein, the term preferably relates to making viral RNA accessible at least partially to the reagents used in step b), preferably to the enzyme or enzymes contacted with the viral RNA in step b). Preferably, releasing comprises disassembling and/or denaturing viral capsid polypeptides and, if present, a viral membrane. In case of samples comprising cells, it preferably is sufficient to disassemble the cytoplasmic membrane of at least part of the cells comprised in said sample. Preferably, releasing comprises contacting the sample with a surfactant, such as Triton X, or a non-ionic surfactant such as Oleth-8. Also, preferably, releasing comprises contacting sample with the aforesaid surfactant Triton X-100 at a concentration of from 0.05% (w/v) to 5% (w/v), preferably of from 0.1% (w/v) to 1% (w/v), more preferably of from 0.15% (w/v) to 0.5% (w/v), most preferably of about 0.2% (w/v). Also preferably, releasing comprises contacting sample with Oleth-8 at a concentration of from 0.10% (w/v) to about 0.40% (w/v), preferably of from about 0.15% (w/v) to about 0.35% (w/v), more preferably of from about 0.20% (w/v) to about 0.30% (w/v), still more preferably about 0.25% (w/v), most preferably 0.25% (w/v) The sample may be heated during the release step, e.g. to at least 90°C for about 5 min. Preferably, the sample is maintained at the same temperature as in the other steps during release, i.e. at a temperature of from 20°C to 45°C, preferably at a temperature of 20°C to 37°C, more preferably at a temperature of about 25°C; as the skilled person will understand, in case the sample is maintained at the same temperature as for the other steps during release, the release step may be comprised in amplification step b). Also, preferably, the viral RNA released is used for the following steps as it is obtained by the aforesaid steps or a combination thereof. Thus, preferably, releasing does not comprise a nucleic acid extraction step. Also, preferably, steps a) and b) are performed in the same reaction tube. Preferably, at least the reaction mixture of step c), preferably the reaction mixtures of steps b) and c), more preferably the reaction mixtures of steps a) to c), comprise at least one RNase inhibitor, preferably an Inhibitor of RNAses A, B, and C, particularly a murine RNase inhibitor, more preferably an inhibitor of RNases A, B, C, 1, and T1, such as the commercially available SUPERase^{™} RNase inhibitor.

The term "amplifying" is used herein in its commonly accepted meaning, i.e. relating to a procedure causing the amount of at least a part of the viral RNA to increase compared to the amount after release of viral RNA from the sample. Methods for amplifying RNA are, in principle, known in the art. Preferred are isothermal methods; thus, preferably, amplifying viral RNA is accomplished via a RNA-dependent RNA polymerase (EC 2.7.7.48), preferably using appropriate primers. More preferably, the viral RNA is amplified by a reverse-transcriptase recombinase polymerase amplification (RT-RPA) reaction. Also, preferably, amplifying further comprises a second amplification step by a T7 RNA polymerase transcription reaction producing amplified viral RNA. RT-RPA and T7 RNA polymerase protocols are known in the art. Appropriate primers are known e.g. from Zhang et al. (2020), cited herein above. Preferably, in case the S gene of SARS-CoV-2 shall be targeted, primers S-RPA-Forward (5'-GAAATTAATACGACTCACTATAGGGAGGTTTCAAACTTTACTT GCTTTACATAGA-3'; SEQ ID NO:1) and S-RPA-Forward (5'-TCCTAGGTTGAAGATAACCCACATAATAAG-3', SEQ ID NO:2) are used; and in case Orf1a shall be targeted, primers Orf1a-RPA-Forward (5'-GAAATTAATACGACTCACTATAGGGCGAAGTTGTAGGAGACATTATACTTAAACC -3'; SEQ ID NO:3) and Orfla-RPA-Reverse (5'-TAGTAAGACTAGAATTGTCTACATAAGCAGC-3', SEQ ID NO:4) are used.

More preferably, in case the N gene of SARS-CoV-2 shall be targeted, primers N-RPA-Forward_v1 (5'-gaaattaatacgactcactatagggTAATCAGACAAGGAACTGATTACAAACATTG-3'; SEQ ID NO:8) and N-RPA-Reverse_v1 (5'- GACTTCCATGCCAATGCGCGACATTCCGAAGA-3', SEQ ID NO:9) or primers N-RPA-Forward_v2 (5'-gaaattaatacgactcactatagggACTAATCAGACAAGGAACTGATTACAAACAT-3'; SEQ ID NO:10) and N-RPA-Reversev2 (5'- CACGTTCCCGAAGGTGTGACTTCCATGCCAAT -3', SEQ ID NO:11) are used; and in case E shall be targeted, primers E-RPA-Forward_v1 (5'-gaaattaatacgactcactatagggGTTAATAGTTAATAGCGTACTTCTTTTTCTTGC-3'; SEQ ID NO:12) and E-RPA-Reverse_v1 (5'- TTAACAATATTGCAGCAGTACGCACACAATC -3', SEQ ID NO:13) or primers E-RPA-Forward_v2 (5'-gaaattaatacgactcactatagggATAGTTAATAGCGTACTTCTTTTTCTTGCTTTC -3'; SEQ ID NO:14) and E-RPA-Reverse_v2 (5'-TACAAGACTCACGTTAACAATATTGCAGCAG-3', SEQ ID NO:15) are used.

The term "Type VI Cas nuclease", also known as C2c2 Type or CasRx Cas nuclease, relates to a Cas nuclease targeting RNA, which, once being activated by a ssRNA sequence being complementary to its crRNA spacer, has unspecific RNase activity and cleaves any RNA in its vicinity. Preferably, the type VI Cas nuclease is a Cas13a nuclease, a Cas13b nuclease, a Cas13c nuclease, or a Cas13d nuclease, more preferably is a Cas13a nuclease. A type VI Cas nuclease can be isolated e.g. from Leptotrichia wadeii, Leptotrichia buccalis, Leptotrichia shahii, Ruminococcus flavefaciens, Bergeyella zoohelcum, Prevotella buccae, or Listeria seeligeri. Preferably, in particular in case step c) is performed at a temperature of 20°C to 37°C, more preferably at a temperature of about 25°C, the type VI Cas nuclease is Cas13a from Leptotrichia buccalis. Also preferably, the type VI Cas nuclease is Cas13b from Capnocytophaga canimorsus Cc5 (CcaCas13b). Moreover, type VI Cas nucleases and expression plasmids for their production are publicly available. Preferably, the type VI Cas nuclease is produced as a His₆-Sumo-Cas nuclease fusion polypeptide and, more preferably, is purified by at least one of His₆-Affinity purification, Sumo affinity purification, and size exclusion chromatography purification. More preferably, the type VI Cas nuclease is produced as a His₆-Sumo-Cas nuclease fusion polypeptide and its purification comprises His₆-Affinity chromatography, Sumo affinity chromatography, and size exclusion chromatography, preferably in the given order.

The term "crRNA", is used herein in relation to a combined crispr RNA (crRNA) of a type VI CRISPR/Cas system. Preferably, the crRNA comprises at least 20, preferably at least 25, more preferably comprises 28 nucleotides complementary to the target sequence. As used herein, the term "complementary", if not otherwise noted, relates to at least 90%, more preferably at least 95%, still more preferably 99% complementarity. Most preferably complementarity relates to 100% complementarity over the aforementioned number of nucleotides. Means and methods for designing crRNAs are known in the art. Preferred crRNase are those disclosed in Zhang et al. (2020), ibd. Thus, preferably, in case Leptotrichia wadeii Cas13a is used and the S gene of a SARS-CoV-2 shall be targeted, the crRNA comprises the sequence of S-crRNA (5'-GAUUUAGACUACCCCAAAAACGAAGGGGACUAAAACGCAGCACCAGCUGUCCA ACCUGAAGAAG-3', SEQ ID NO:5), and in case the Orf1a shall be targeted, the crRNA comprises the sequence of Orfla-crRNA (5'-GAUUUAGACUACCCCAAAAACGAAGGGGACUAAAACCCAACCUCUUCUGUAAU UUUUAAACUAU-3', SEQ ID NO:6).Most, preferably, in case Leptotrichia wadeii Cas13a is used and the N gene of a SARS-CoV-2 shall be targeted, the crRNA comprises the sequence of N-crRNA (5'-GAUUUAGACUACCCCAAAAACGAAGGGGACUAAAACGAACGCUGAAGCGCUGG GGGCAAAUUGU-3', SEQ ID NO:16), and in case the E gene shall be targeted, the crRNA comprises the sequence of E-crRNA (5'-GAUUUAGACUACCCCAAAAACGAAGGGGACUAAAACUCGAAGCGCAGUAAGGA UGGCUAGUGUA-3', SEQ ID NO: 17).

The term "reporter RNA" in principle, relates to any polynucleotide comprising at least one pair of ribonucleotides connected via a covalent bond cleavable by a Cas RNase activity, the cleavage of which can be determined. Preferably, the reporter RNA comprises at least a first label, preferably attached towards one of the ends of said reporter RNA, the term "towards the end" relating to an attachment within 10 nucleotides from an end, preferably within 5 nucleotides from an end, more preferably at the terminal nucleotide. Further, the term "attached", as used herein, includes any type of attachment, covalent or non-covalent, which is sufficiently stable to allow detection of the association of the label and the reporter RNA or fragment thereof. Thus, preferably, the label or labels preferably is/are linked to the reporter RNA by non-covalent bonds and the two molecules have a dissociation constant of at most 10⁻⁶ mol/l, more preferably of at most 10⁻⁷ mol/l, most preferably at most 10⁻⁸ mol/l. Also, preferably, the reporter RNA and the label or labels are covalently connected. More preferably, the reporter RNA comprises a first and a second label, wherein the first label is attached towards the 5' end of the reporter RNA and the second label is attached towards the 3' end of the reporter RNA. Preferably, a dye is the first label and a quencher is the second label; appropriate dye/quencher pairs are known in the art. More preferably, at least one of said first and second labels is an affinity label, i.e. a label with affinity for a binding partner; in such case, detecting cleavage of said reporter RNA preferably comprises binding of said affinity label to a solid surface. Suitable affinity pairs are known in the art, e.g. the streptavidin/biotin affinity pair. Thus, one of the first and second labels may e.g. be biotin. Also, preferably, the second label is a second affinity label non-identical to the first affinity label and/or is a detectable label, in particular a dye. More preferably, the second label is a fluorescein, preferably 6-carboxyfluorescein or fluorescein; antibodies specifically recognizing fluoresceins are available, thus said label may be detected optically or via an immunological detection method. Preferably, the second label is a chemical group specifically detected by antibodies conjugated to gold particles, as Exemplified in the lateral flow assay described herein in the Examples. Preferably, the reporter RNA has the sequence of the lateral-flow-reporter described by Zhang et al. (2020), ibd, (5'-6-FAM-mArArUrGrGrCmAmArArUrGrGrCmA-Bio-3', SEQ ID NO:7, with 6-FAM: 6-carboxyfluorescein, Bio: Biotin; rX (X=A, U, G, or C): ribonucleotides; mA: 2'-O-methyl-adenosine). In an also preferred embodiment, the reporter RNA has the sequence of the lateral-flow-reporter described by Kellner et al. (2019), Nature Protocols 14:2986, (5'-6-FAM/rUrUrUrUrUrU/3'-Bio (SEQ ID NO:24)) or 5'-TEX615-T*A*rArUG*C*-3IAbRQSp-3', SEQ ID NO:26, with TEX615:Texas Red^{®}, * following a base indicates a phosphorothioate bond to the following nucleotide, and IAbRQSp is a Iowa Black^{®} RQ-Sp). Preferably, the reporter RNA, in particular the reporter RNA having SEQ ID NO:7, is present in the assay at an amount of up to 100 pmol, more preferably up to 50 pmol, still more preferably up to 20 pmol, most preferably up to 10 pmol, per assay mixture. Also preferably, the reporter RNA, in particular the reporter RNA having SEQ ID NO:7, is present in the assay at an amount of from about 10 pmol to 100 pmol, more preferably of from about 10 pmol to 50 pmol, still more preferably of from about 10 pmol to 20 pmol, most preferably of about 10 pmol, per assay mixture. As is understood by the skilled person from the description herein, the nucleotide sequence of the reporter RNA is of minor importance, since the activity to be detected is nonspecific RNase activity caused by the presence of an RNA hybridizing to the crRNA.

The term "detecting cleavage of a reporter RNA" relates to assessing to which extent the reporter RNA was cleaved, wherein said extent may be any fraction of from 0% to 100% of the reporter RNA present in the assay mixture. Suitable methods include all methods enabling establishing whether at least one covalent backbone bond was cleaved by an RNase activity in reporter molecules. Thus, the reporter RNA may e.g. be a mixed DNA/RNA oligonucleotide comprising ribonucleotides near the center of the molecule, such that cleavage by an RNase produces smaller fragments, which can be detected. In case the reporter RNA comprises two labels, RNase activity on such a reporter RNA causes the first label to become separated from the second label, which separation can be detected. Preferably, said detection comprises separation of the first from the second label, e.g. by diffusion. E.g., preferably, in case the reporter RNA comprises a dye and a quencher, increased radiation emission by the dye may be detected. In case the reporter RNA comprises at least one affinity label, detection of cleavage may be performed by lateral flow diffusion, in particular as described by Zhang et al (2020), ibd., and herein in the Examples. As is understood from the description herein, detecting cleavage of reporter RNA is detecting presence of an RNA hybridizing to the crRNA, i.e. viral RNA and/or amplified viral RNA. More preferably, detecting increased cleavage of reporter RNA compared to a negative control is detecting presence of an RNA hybridizing to the crRNA, i.e. viral RNA and/or amplified viral RNA, and therefore is detecting an RNA virus. Detection of cleavage of reporter RNA may be qualitative, semiquantitative, or quantitative; preferably, detection of cleavage of reporter RNA is qualitative or semiquantitative.

Advantageously, it was found in the work underlying the present invention that detection of RNA viruses, in particular coronaviruses, is possible by a method not requiring technical means for temperature adjustment. In an aspect, the method can be performed using only two temperatures, namely the temperature of boiling water, and ambient temperature, e.g. standard temperature, or the body temperature of a mammal. This is particularly advantageous for applications in less developed countries, in which technical instrumentation may be scarce. In a further aspect, the complete method can be performed at the latter temperature. Furthermore, it was found that release of the viral RNA and its amplification can be performed in the same test tube, further simplifying the method.

The definitions made above apply mutatis mutandis to the following. Additional definitions and explanations made further below also apply for all embodiments described in this specification mutatis mutandis.

The present invention also relates to a method for detecting a target RNA and/or a target DNA in a sample of a subject, the method comprising the steps of
a) contacting said sample with a Type V Cas nuclease, a Type V crRNA targeting said target DNA, a type VI Cas nuclease, a Type VI crRNA targeting said target RNA, a reporter RNA and a reporter DNA; and
b) detecting cleavage of said reporter RNA, thereby detecting said target RNA; and/or detecting cleavage of said reporter DNA, thereby detecting said target DNA.

The term "target RNA", as used herein, preferably relates to a polyribonucleotide of interest, suspected or known to be present in a sample. Preferably, the target RNA comprises at least 20, more preferably at least 25, even more preferably at least 28 nucleotides. Thus, in case a longer RNA shall be detected, the target RNA may be said RNA as such, or may be a fragment thereof having the aforesaid length. In accordance, the term "at least a fragment" may relate to a subportion of an polynucleotide, but also to the full-length polynucleotide; preferably, said fragment comprises at least one nucleic acid sequence allowing specific detection of said target RNA, more preferably a specific nucleic acid sequence, even more preferably a nucleic acid sequence of at least 20 nucleotide, more preferably at least 25 nucleotides, even more preferably at least 28 nucleotides which is known or expected to be unique in said sample. Thus, preferably, the target RNA does not consist of low-complexity sequences, such as short repeat sequences. The target RNA may, in principle, be any type of RNA, such as double-stranded or single-stranded RNA, or linear or circular RNA. Preferably, the target RNA is an RNA being of diagnostic or therapeutic interest. Thus, preferably, the target RNA is at least a fragment of a genome of an RNA virus or viroid, preferably of an RNA virus. Also preferably, the target RNA is at least a fragment of an RNA of a pathogen, e.g. an RNA encoded by a pathogen as specified herein below, such as an RNA expressed from a promoter and/or one or more gene(s) of a virus, a bacterium, or a eukaryotic pathogen. Also preferably, the target RNA is at least a fragment of an endogenous RNA, i.e. an RNA expressed by cells of the subject, preferably cells known or suspected to be comprised in the sample. Endogenous RNAs are known to the skilled person and include in particular mRNAs, miRNAs, tRNAs, ribosomal RNAs (rRNAs), snRNAs, snoRNAs, and siRNAs; preferably, the endogenous RNA is an mRNA. As the skilled person understands, the target RNA may also be allele-specific, i.e. indicative of a specific allele of a gene. Also, the target RNA may be cell-type-specific, e.g. in case at least a fragment of an RNA, e.g. mRNA, expressed only by (a) certain type(s) of cell(s) is used as target RNA. Thus the target RNA may be at least a fragment of a cell-type specific RNA, e.g. a cancer-specific RNA, such as an mRNA of a cancer-specific marker. As the skilled person understands, specificity may also be achieved by a specific combination of sample and target RNA, such as testing for melanocyte markers in a blood sample, e.g. in screening for circulating melanoma cells.

The term "target DNA", as used herein, preferably relates to a polydeoxyribonucleotide of interest, suspected or known to be present in a sample or producible therefrom. The target DNA may, preferably, be any type of DNA, such as double-stranded or single-stranded DNA, or linear or circular DNA; preferably, the target DNA comprises at least 20, more preferably at least 21, even more preferably at least 24 nucleotides. Preferably, the target DNA is at least a fragment of a DNA of a cell or virus, e.g. of a genomic DNA, an organellar DNA, a plasmid, and the like. More preferably, the target DNA is a reverse transcript of an RNA, preferably an RNA as specified herein above. Thus, the above definitions of the target RNA apply, mutatis mutandis, to the target DNA as well. Preferably, the target DNA is at least a fragment of a a reverse transcript of an RNA comprised in said sample, the term "RNA comprised in said sample" including any and all RNA molecules comprised in the sample, including an RNA optionally added to the sample before, during, or after extraction (spiking). More preferably, the RNA comprised in the sample is an RNA according to the specification of the target RNA herein above. More preferably, the target DNA is a reverse transcript of an endogenous RNA as specified herein above, more preferably of an mRNA, preferably of a housekeeping gene of the subject. Preferably, the target DNA is a control DNA, in particular a control for sample extraction, sample reverse transcription, and/or absence of RNases.

The term "pathogen" is understood by the skilled person and includes, preferably, any and all organisms, viruses, and viroids causing disease in a non-identical organism. Thus, the pathogen preferably is a bacteriophage, a plant pathogen, a pathogen of livestock, laboratory, or companion animal, more preferably of a mammal, most preferably of a human.

The term "Type V Cas nuclease", also known as C2c1 Type or Cpf1 Cas nuclease, relates to a Cas nuclease targeting DNA, preferably ssDNA, which, once being activated by a DNA sequence being complementary to its Type V crRNA spacer, has unspecific DNase activity and cleaves any DNA, preferably ssDNA, in its vicinity. Preferably, the type V Cas nuclease is a Cas12a nuclease, more preferably is Cas12a from Lachnospiraceae bacterium ND2006 (LbCas12a)(Chen et al. (2018), Science 360(6387):436). A type V Cas nuclease can also be isolated e.g. from Francisella novicida, Acidaminococcus sp., other Lachnospiraceae sp., Alicyclobacillus acidoterrestris, Acidaminococcus sp. BV3L6, or Prevotella sp. Preferably, in particular in case step a) is performed at a temperature of 20°C to 42°C, more preferably at a temperature of about 25°C, the type V Cas nuclease is LbCas12a. Moreover, type V Cas nucleases and expression plasmids for their production are publicly available. Preferably, the type V Cas nuclease is produced as a His₆-Sumo-Cas nuclease fusion polypeptide and, more preferably, is purified by at least one of His₆-Affinity purification, Sumo affinity purification, and size exclusion chromatography purification. More preferably, the type V Cas nuclease is produced as a His₆-Sumo-Cas nuclease fusion polypeptide and its purification comprises His₆-Affinity chromatography, Sumo affinity chromatography, and size exclusion chromatography, preferably in the given order.

As specified herein above, the term "crRNA", unless specified otherwise, relates to a combined crispr RNA (crRNA) of a type VI CRISPR/Cas system, i.e. a Type VI crRNA. In accordance, the term "Type V crRNA" is used herein in relation to a combined crispr RNA (crRNA) of a type V CRISPR/Cas system. As the skilled person is aware of, a Type V crRNA preferably comprises a protospacer adjacent motif (PAM) sequence. Preferably, the Type V crRNA comprises at least 20, preferably at least 21, more preferably comprises 24 nucleotides complementary to the target sequence. Means and methods for designing Type V crRNAs are known in the art. Thus, preferably, in case Cas12 is used and a reverse transcript of an RNaseP mRNA is the target DNA, the Type V crRNA comprises the sequence of SEQ ID NO:23..

The term "reporter DNA", preferably, relates to any DNA polynucleotide comprising at least one pair of deoxyribonucleotides connected via a covalent bond cleavable by a Cas12 DNase activity, the cleavage of which can be determined. Preferably, the reporter DNA comprises at least one label, preferably attached towards one of the ends of said reporter DNA, the terms "towards the end" and "attached" having, mutatis mutandis, the meaning as specified herein above in the context of the reporter RNA. Thus, preferably, the label or labels preferably is/are linked to the reporter DNA by non-covalent bonds and the two molecules have a dissociation constant of at most 10⁻⁶ mol/l, more preferably of at most 10⁻⁷ mol/l, most preferably at most 10⁻⁸ mol/l. Also, preferably, the reporter DNA and the label or labels are covalently connected. As used herein, the terms first and second label are preferably used in connection with the reporter RNA as specified herein above; thus, in the context of the target DNA, the terms third and second label are preferably used. Thus, preferably, the reporter DNA comprises a third and a fourth label, wherein the third label is attached towards the 5' end of the reporter DNA and the fourth label is attached towards the 3' end of the reporter DNA. Preferably, a dye is the third label and a quencher is the fourth label; appropriate dye/quencher pairs are known in the art. More preferably, at least one of said third and fourth labels is an affinity label, i.e. a label with affinity for a binding partner; in such case, detecting cleavage of said reporter DNA preferably comprises binding of said affinity label to a solid surface. Suitable affinity pairs are known in the art, e.g. the streptavidin/biotin affinity pair. Thus, one of the third and fourth labels may e.g. be biotin. Also, preferably, the fourth label is a fourth affinity label non-identical to the third affinity label and/or is a detectable label, in particular a dye. More preferably, the fourth label is a digoxigenin; antibodies specifically recognizing digoxigenin are available, thus said label may be detected optically or via an immunological detection method. Preferably, the fourth label is a chemical group specifically detected by antibodies conjugated to gold particles, as Exemplified in the lateral flow assay described herein in the Examples. Preferably, the reporter DNA has the sequence of SEQ ID NO:18, which may be provided e.g. as reporter DNA according to any one of SEQ ID NOS: 19-22. Preferably, the first to fourth labels, as referred to herein, are mutually non-identical. It may, however, also be envisaged that the reporter RNA and the reporter DNA have the same affinity label, e.g. fluorescein, e.g. in applications such as lateral flow detection.

As the skilled person understands, since the nuclease activities of Type V and Type VI Cas nucleases are, once activated, non-sequence specific, the reporter RNA and/or reporter DNA, e.g. as specified herein, may in principle be used for detection of any target RNA and/or target DNA.

The term "subject", preferably relates to a living organism, more preferably a multicellular organism. The subject preferably is a plant, more preferably a crop plant. More preferably, the subject is a vertebrate, still more preferably a mammal, even more preferably a livestock, laboratory, or companion animal, most preferably is a human.

The method for detecting a target RNA and/or a target DNA preferably is an in vitro method. Moreover, it may comprise steps in addition to those specifically mentioned; e.g. a further step preferably preceding step a) may relate to extracting and/or purifying RNA and/or DNA from said sample; also, the method may be comprised in a method for detecting an RNA virus in a sample as specified elsewhere herein, in particular by using an RNA from the RNA virus as target RNA, and using e.g. a reverse transcript of a housekeeping mRNA of the subject as the target DNA.

Thus, the present invention preferably relates to a method for detecting an RNA virus in a sample, comprising the steps of
i) releasing RNA from said sample;
ii) amplifying at least parts of the RNA comprised in said sample via a DNA intermediate;
iii) detecting a target RNA and a target DNA in the reaction mixture of step ii) according to the method for detecting a target RNA and/or a target DNA according to the present invention; and
iv) thereby detecting said RNA virus.

The method for detecting a target RNA and/or a target DNA may comprise detecting a target RNA, detecting a target DNA, or detecting a target RNA and a target DNA, preferably comprises detecting a target RNA and a target DNA. In view of the description herein above, the skilled person understands that, in relation to the polynucleotides comprised in the sample, said method preferably is a method for detecting (i) two non-identical RNAs of interest or (ii) an RNA and a DNA of interest. In accordance, the method for detecting a target RNA and/or a target DNA is applicable in each case where detection of two non-identical RNAs or of an RNA and a DNA is desirable.

Preferably, in the method for detecting a target RNA and/or a target DNA, at least one, preferably one, of the target RNA and target DNA, preferably the target DNA, is a control polynucleotide, in particular a control for sample extraction, sample reverse transcription, and/or absence of RNases. Thus, preferably, the target RNA or the target DNA is or is derived from a polynucleotide known to be present in the sample, more preferably is an RNA present in abundance in at least one cell type known to be present in the sample. Preferably, the target RNA or the target DNA is or is derived from an mRNA of a housekeeping gene of at least one cell type known to be present in the sample, more preferably of essentially all cell types suspected to be present in said sample. Most preferably the target RNA or the target DNA is or is derived from an mRNA of a general housekeeping gene of essentially all nucleated cells of said subject, such as RNaseP mRNA in a mammal, in particular a human.

The method for detecting a target RNA and/or a target DNA may comprise additional steps of extracting and/or purifying polynucleotides from the sample, preferably as specified herein above; the method may also comprise one or more steps of concentrating or diluting polynucleotides comprised in the sample.

In case the polynucleotides of interest are known to be present in the sample in high abundance, the method for detecting a target RNA and/or a target DNA may be applied in the absence of an amplification step. Preferably, in particular in case at least one polynucleotide of interest is known or suspected to be present at low abundance in the sample, polynucleotides comprised in the sample are amplified. Said amplification may be non-sequence specific, e.g. by using non-sequence specific primers such as oligo(dT) primers, preferably is sequence specific for at least one polynucleotide of interest. Preferably, amplification is effected as specified herein above. Thus, preferably, amplification comprises at least one step of creating a DNA intermediate of at least one RNA of interest or fragment thereof, e.g. by reverse transcription. Preferably, this step is used for at least one RNA in case two non-identical RNAs shall be detected. In a further step, RNA may be produced from said DNA, e.g. by T7-transcription, preferably as specified herein above and/or in the Examples. As the skilled person understands, it may be sufficient to amplify a fragment of a polynucleotide of interest such as to produce or amplify a target DNA and/or a target RNA.

The method for detecting a target RNA and/or a target DNA, as is understood by the skilled person, is a multiplex method in that it allows detection of a target RNA and a target DNA, i.e. of two polynucleotides of interest. The method may, however, be further multiplexed: In an embodiment, the Type V crRNA and/or Type VI crRNA may be selected as to be specific for a generic group of target RNAs or target DNAs; as a non-limiting example, a crRNA may be selected to bind to a conserved nucleic acid sequence comprised in all SARS viruses; as will be understood, in such case, detecting cleavage of the reporter DNA or reporter RNA is indicative of at least one member of the generic group being present. Similarly, the reporter DNA and/or reporter RNA may be selected such as to be indicative of a generic group of polynucleotides. In a further embodiment, step a) may comprise contacting said sample with a multitude of Type V crRNAs and/or Type VI crRNAs; in such case, detecting cleavage of said reporter RNA and/or of said reporter DNA would be indicative that a target of at least one of said Type V crRNAs and/or Type VI crRNAs was present in the sample. Thus, by way of example, the method may be used to test a sample for the presence of at least one member of a predefined set of viruses and for the presence of at least one member of a predefined set of bacteria, or to test a sample for the presence of at least one member of two predefined sets of viruses, e.g. SARS viruses and influenza viruses.

The term "detecting cleavage" has been specified herein above in the context of a reporter RNA; the same applies mutatis mutandis to reporter DNAs.

The present invention also relates to a type V Cas nuclease and/or a type VI Cas nuclease for use in diagnosing an infection with an RNA virus in a subject, preferably according to the method for detecting a target RNA and/or a target DNA or a method for detecting an RNA virus in a sample according to the present invention.

The present invention also relates to a method for diagnosing an infection with an RNA virus in a subject, comprising
a) detecting an RNA virus in a sample of said subject by a method of the present invention; and
b) in case an RNA virus is detected in step a), diagnosing an infection with an RNA virus in said subject.

The present invention further relates to a use of a type VI Cas nuclease, preferably a Cas13a nuclease, a Cas13b nuclease, a Cas13c nuclease, or a Cas13d nuclease, for detecting an RNA virus, preferably according to the method for detecting an RNA virus described herein above.

The present invention also relates to a use of a type VI Cas nuclease, preferably a Cas13a nuclease, a Cas13b nuclease, a Cas13c nuclease, or a Cas13d nuclease, for the manufacture of a diagnostic composition or device for the detection of an RNA Virus, preferably according to the method for detecting an RNA virus described herein above.

Furthermore, the present invention relates to a method for diagnosing an infection with an RNA virus in a subject, comprising
a) detecting an RNA virus in a sample of said subject by the method for detecting an RNA virus described herein above; and
b) in case an RNA virus is detected in step a), diagnosing an infection with an RNA virus in said subject.

The term "diagnosing", as used herein, means assessing whether a subject suffers from an RNA virus infection, or not. As will be understood by those skilled in the art, such an assessment is usually not intended to be correct for all (i.e. 100%) of the subjects to be identified. The term, however, requires that preferably a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the subjects of a population can be properly identified by the method of the present invention. Diagnosing according to the present invention may include applications of the method in monitoring, confirmation, and sub-classification of the RNA virus infection.

The present invention also relates to a type VI Cas nuclease for use in diagnosing an infection with an RNA virus in a subject, preferably according to the method for detecting an RNA virus described herein above.

In view of the above, the following embodiments are specifically envisaged:
1. A method for detecting an RNA virus in a sample, preferably a saliva or sputum sample, comprising the steps of
   a) releasing viral RNA from said sample;
   b) amplifying at least parts of the viral RNA comprised in said sample;
   c) contacting the amplified viral RNA of step a) with a Type VI Cas nuclease, a crRNA, and a reporter RNA; and
   d) detecting cleavage of said reporter RNA, thereby detecting said RNA virus.
2. The method of embodiment 1, wherein wherein steps a) and b) are performed in the same reaction tube.
3. The method of embodiment 1 or 2, wherein said step a) comprises contacting said sample with a surfactant, preferably a non-ionic surfactant.
4. The method of any one of embodiments 1 to 3, wherein said step a) comprises contacting said sample with said surfactant at a concentration of from 0.05% (w/v) to 5% (w/v).
5. The method of any one of embodiments 1 to 4, wherein steps b) to c), preferably all steps, are performed at a temperature of from 20°C to 45°C.
6. The method of any one of embodiments 1 to 5, wherein steps b) to c), preferably all steps, are performed at a temperature of 20°C to 37°C.
7. The method of any one of embodiments 1 to 6, wherein steps b) to c), preferably all steps, are performed at a temperature of about 25°C.
8. The method of any one of embodiments 1 to 7, wherein said step a) comprises contacting the viral RNA with a primer and an RNA polymerase, preferably an RNA-dependent RNA polymerase.
9. The method of any one of embodiments 1 to 8, wherein said step a) comprises amplifying said viral RNA by reverse-transcriptase recombinase polymerase amplification (RT-RPA), preferably comprising contacting said viral RNA with MuLV reverse transcriptase.
10. The method of any one of embodiments 1 to 9, wherein said RNA virus is SARS-CoV-2, SARS-CoV-1, or MERS.
11. The method of any one of embodiments 1 to 10, wherein said reporter RNA comprises at least a first label, preferably attached towards one of the ends of said reporter RNA.
12. The method of any one of embodiments 1 to 11, wherein said reporter RNA comprises a first and a second label, wherein the first label is attached towards the 5' end of the reporter RNA and the second label is attached towards the 3' end of the reporter RNA.
13. The method of any one of embodiments 1 to 12, wherein at least one of said first and second label is an affinity label.
14. The method of any one of embodiments 1 to 13, wherein detecting cleavage of said reporter RNA comprises detecting separation of the first label from the second label.
15. The method of any one of embodiments 1 to 14, wherein detecting cleavage of said reporter RNA comprises binding of said affinity label to a solid surface.
16. The method of any one of embodiments 1 to 15, wherein said type VI Cas nuclease is a Cas13a nuclease, a Cas13b nuclease, a Cas13c nuclease, or a Cas13d nuclease, preferably is a Cas13a nuclease, more preferably a Cas13a nuclease from Leptotrichia wadeii (LwaCas13a) or from Leptotrichia buccalis (LbuCas13a).
17. The method of any one of embodiments 10 to 16, wherein said crRNA binds specifically within the Orf1a open reading frame or in the S open reading frame.
18. The method of any one of embodiments 1 to 17, wherein in steps a) and b), the viral RNA is further contacted to an inhibitor of RNases A, B, C, 1, and T1.
19. Use of a type VI Cas nuclease, preferably a Cas13a nuclease, a Cas13b nuclease, a Cas13c nuclease, or a Cas13d nuclease, for detecting an RNA virus, preferably according to the method of any one of embodiment s 1 to 18.
20. A type VI Cas nuclease for use in diagnosing an infection with an RNA virus in a subject, preferably according to the method according to any one of embodiments 1 to 19.
21. The type VI Cas nuclease for use of embodiment 20, wherein said RNA virus is a SARS-CoV-2, SARS-CoV-1, or MERS.
22. A method for diagnosing an infection with an RNA virus in a subject, comprising
   a) detecting an RNA virus in a sample of said subject by the method of any one of embodiments 1 to 19; and
   b) in case an RNA virus is detected in step a), diagnosing an infection with an RNA virus in said subject.
23.A method for detecting a target RNA and/or a target DNA in a sample of a subject, the method comprising the steps of
   a) contacting said sample with a Type V Cas nuclease, a Type V crRNA targeting said target DNA, a type VI Cas nuclease, a Type VI crRNA targeting said target RNA, a reporter RNA and a reporter DNA; and
   b) detecting cleavage of said reporter RNA, thereby detecting said target RNA; and/or detecting cleavage of said reporter DNA, thereby detecting said target DNA.
24. The method of embodiment 23, wherein said target RNA is at least a fragment of a genome of an RNA virus, of a pathogen RNA, or of an endogenous RNA of said subject, preferably an mRNA.
25. The method of embodiment 23 or 24, wherein said target DNA is at least a fragment of a DNA comprised in said sample of said subject.
26. The method of any one of embodiments 23 to 25, wherein said target DNA is a reverse transcript of an RNA comprised in said sample, preferably of an endogenous RNA, more preferably of an RNA of a housekeeping gene of said subject.
27. The method of any one of embodiments 23 to 26, wherein said method further comprises extracting RNA and/or DNA from said sample before step a).
28. The method of any one of embodiments 23 to 27, wherein said target DNA is a control DNA for controlling extraction.
29. The method of any one of embodiments 23 to 28, wherein said target DNA is a control DNA produced from said sample via reverse transcription and is for controlling extraction, reverse transcription and/or absence of RNases.
30. The method of any one of embodiments 23 to 29, wherein said target RNA is at least a fragment of a genome of an RNA virus and wherein said target DNA is a reverse transcript of an endogenous transcript of said subject, preferably of a transcript of a housekeeping gene of said subject.
31. The method of any one of embodiments 23 to 30, wherein said reporter RNA is a single-stranded RNA.
32. The method of any one of embodiments 23 to 31, wherein said reporter RNA comprises a first and a second label, wherein the first label is attached towards the 5' end of the reporter RNA and the second label is attached towards the 3' end of the reporter RNA.
33. The method of any one of embodiments 23 to 32, wherein said reporter DNA is a single-stranded DNA.
34. The method of any one of embodiments 23 to 33, wherein said reporter DNA comprises a third and a fourth label, wherein the third label is attached towards the 5' end of the reporter DNA and the fourth label is attached towards the 3' end of the reporter DNA.
35. The method of any one of embodiments 23 to 34, wherein at least one of said first and second labels is an affinity label and/or wherein at least one of said third and fourth labels is an affinity label.
36. The method of any one of embodiments 23 to 35, wherein at least one of said first and second labels is a quencher and/or wherein at least one of said third and fourth labels is a quencher.
37. The method of any one of embodiments 23 to 36, wherein detecting cleavage of said reporter RNA comprises detecting separation of the first label from the second label and/or wherein detecting cleavage of said reporter DNA comprises detecting separation of the second label from the fourth label.
38. The method of any one of embodiments 23 to 37, wherein the first to fourth labels are mutually non-identical.
39. The method of any one of embodiments 23 to 38, wherein said type VI Cas nuclease is a Cas13a nuclease, a Cas13b nuclease, a Cas13c nuclease, or a Cas13d nuclease, preferably is a Cas13a nuclease, more preferably a Cas13a nuclease from Leptotrichia wadeii (LwaCas13a) or from Leptotrichia buccalis (LbuCas13a) or from Capnocytophaga canimorsus Cc5 (CcaCas13b).
40. The method of any one of embodiments 23 to 39, wherein said type V Cas nuclease is a Cas12a nuclease, preferably from Lachnospiraceae bacterium ND2006 Cas12a (LbCas12a) or Acidaminococcus sp. BV3L6 (AsCas12a) or a Cas12b nuclease, preferably from Alicyclobacillus acidiphilus (AaCas12b).
41. A method for detecting an RNA virus in a sample, comprising the steps of
   i) releasing RNA from said sample;
   ii) amplifying at least parts of the RNA comprised in said sample via a DNA intermediate;
   iii) detecting a target RNA and a target DNA in the reaction mixture of step ii) according to the method according to any one of embodiments 23 to 40; and
   iv) thereby detecting said RNA virus.
42. The method of embodiment 41, wherein said target RNA is an RNA comprised in the genome of the RNA virus.
43. The method of embodiment 41 or 42, wherein said Type VI crRNA binds specifically within the Orf1a open reading frame or in the S open reading frame.
44. The method of any one of embodiments 41 to 43, wherein said target DNA is a reverse transcript of a transcript of said subject, preferably an endogenous transcript, more preferably of a transcript of a housekeeping gene of said subject, most preferably of an RNase P transcript.
45. The method of any one of embodiments 41 to 44, wherein step ii) comprises contacting the viral RNA with a primer and an RNA polymerase, preferably an RNA-dependent RNA polymerase.
46. The method of any one of embodiments 41 to 45, wherein said step ii) comprises amplifying said viral RNA by reverse-transcriptase recombinase polymerase amplification (RT-RPA).
47. The method of any one of embodiments 41 to 46, wherein said RNA virus is SARS-CoV-2, SARS-CoV-1, or MERS.
48. A type V Cas nuclease and/or a type VI Cas nuclease for use in diagnosing an infection with an RNA virus in a subject, preferably according to the method according to any one of embodiments 23 to 47.
49. A method for diagnosing an infection with an RNA virus in a subject, comprising
   a) detecting an RNA virus in a sample of said subject by a method of any one of embodiments 23 to 47; and
   b) in case an RNA virus is detected in step a), diagnosing an infection with an RNA virus in said subject.

All references cited in this specification are herewith incorporated by reference with respect to their entire disclosure content and the disclosure content specifically mentioned in this specification.

### Figure Legends

Fig. 1: Schematic representation of the improved assay protocol.

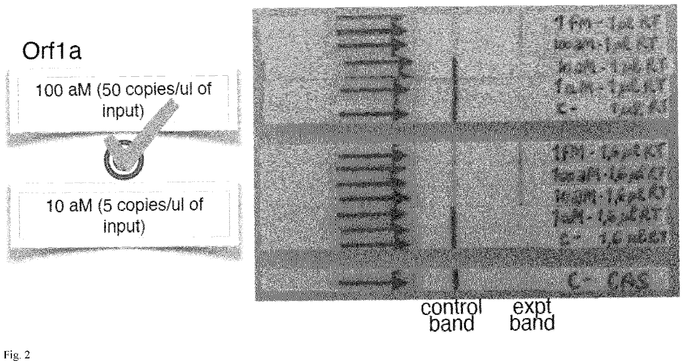

Fig. 2: Results of a SHERLOCK assay targeting SARS-CoV-2 Orfla, modified according to the invention; 5 copies / µl of input can be detected.

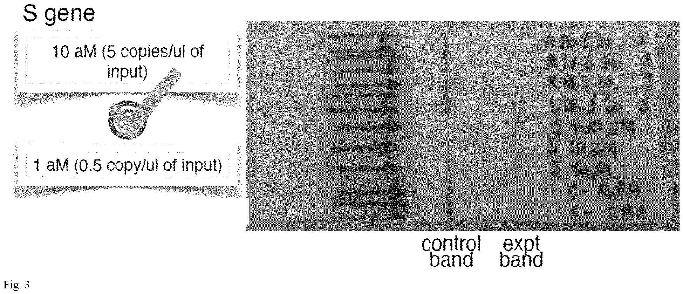

Fig. 3: Results of a SHERLOCK assay targeting the SARS-CoV-2 S gene, modified according to the invention; 0.5 copies / µl of input can be detected.
Fig. 4: Comparison of Superase as RNase inhibitor to murine RNase inhibitor in a SHERLOCK assay; SUPERase reduces background and improves differentiation.
Fig. 5: Optimization of reporter RNA amount; down to 10 pmol can be used without loss of sensitivity.
Fig. 6: comparison of the results of prior art (qRT-PCR) testing for SARS-CoV-2 to the assay of the present invention; results are identical, except for samples close to the detection limit.

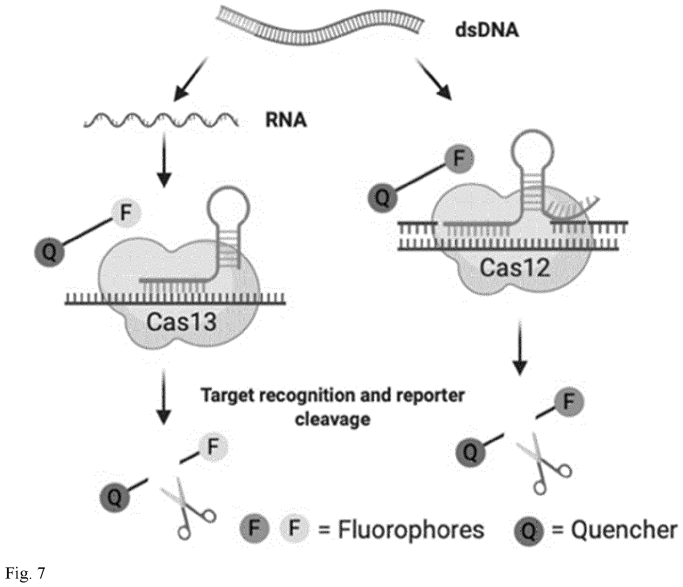

Fig. 7: Schematic representation of a Type VI/Type V Cas multiplex assay, exemplified as a fluorescence assay; dsDNA and RNA may be produced e.g. from RNA in a sample, e.g. by RPA as described in the Examples. Target RNA activates Cas13, which in turn cleaves reporter RNA; target DNA activates Cas12, which in turn cleaves reporter DNA; cleavage of the reporter separates the quencher from the respective fluorophore.

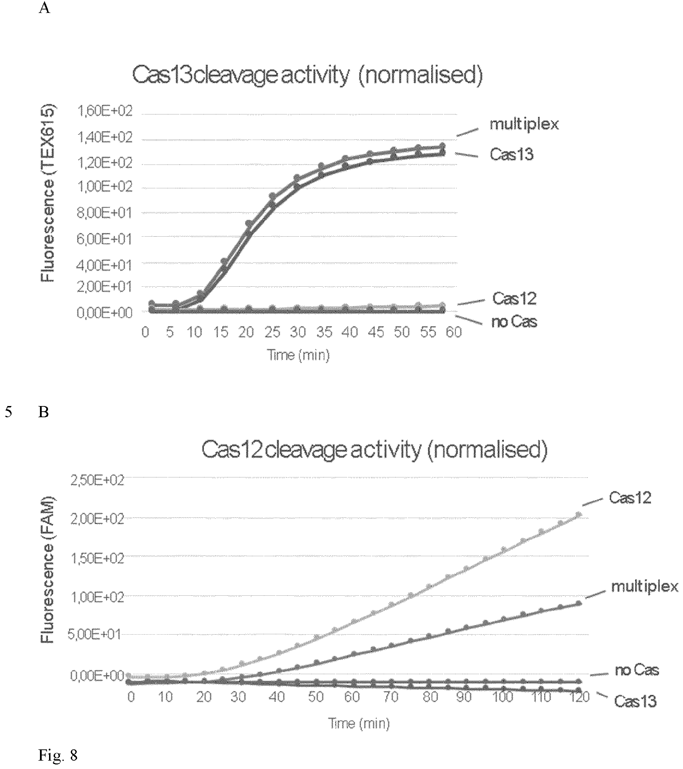

Fig. 8: Specificity of detection by Cas12 and Cas13: A) Fluorescence of TEX615 reporter RNA (SEQ ID NO:26) overtime after incubation with Cas 12, Cas 13, Cas12 crRNA, Cas13 crRNA, and reporter RNA ("multiplex") or with Cas 12, Cas12 crRNA, and reporter RNA ("Cas12") or with Cas 13, Cas13 crRNA, and reporter RNA ("Cas13") or with Cas12 crRNA, Cas13 crRNA, and reporter RNA ("No Cas"); B) Fluorescence of FAM reporter DNA (SEQ ID NO:19) over time after incubation with Cas 12, Cas 13, Cas12 crRNA, Cas13 crRNA, and reporter DNA ("multiplex") or with Cas 12, Cas12 crRNA, and reporter DNA ("Cas12") or with Cas 13, Cas13 crRNA, and reporter DNA ("Cas13") or with Cas12 crRNA, Cas13 crRNA, and reporter DNA ("No Cas").

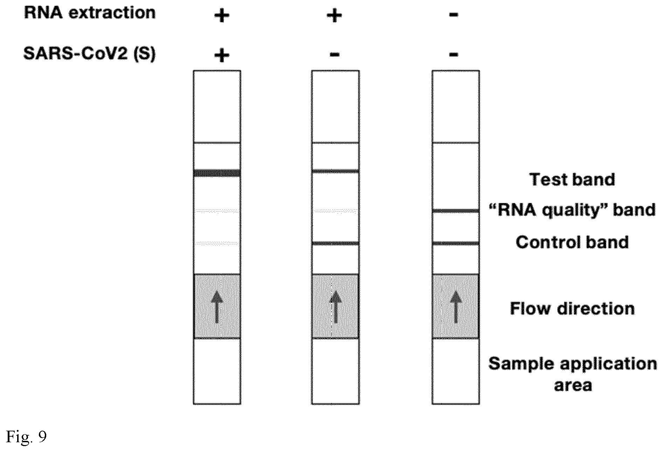

Fig. 9: Expected result of lateral flow assays with HybriDetect 2T sticks: for RNA extraction and SARS-CoV2, + indicates the result being positive, i.e. successful RNA extraction and presence of SARS-CoV-2, respectively.

The following Examples shall merely illustrate the invention. They shall not be construed, whatsoever, to limit the scope of the invention.

### Example 1: Improvements of SHERLOCK assays for SARS-CoV-2

The assay for SARS-CoV-2 was performed essentially by the assay proposed by Zhang et al. (2020), cited above, with the following modifications:
step (a) lyse patient material directly to liberate RNA: add TritonX to 0.2% final & boil (5 min);
step (b) isothermal amplification of viral RNA: add 1,8 µL of patient material to pre-made tube with reagents for reverse transcription and isothermal amplification of viral RNA (25 min);
step (c) guide-specific cas13 binding and reporter cleavage: add 1 µL from previous reaction; T7 pol produce RNA that can be detected by Cas13: reporter guide: 5'-/56-FAM/mArArUrGrGrCmAmArArUrGrGrCmA/3Bio/-3' (SEQ ID NO:7) (30min);
step (e) lateral flow readout (dip - strip): anti-bio and anti-fluor antibodies (2 min).

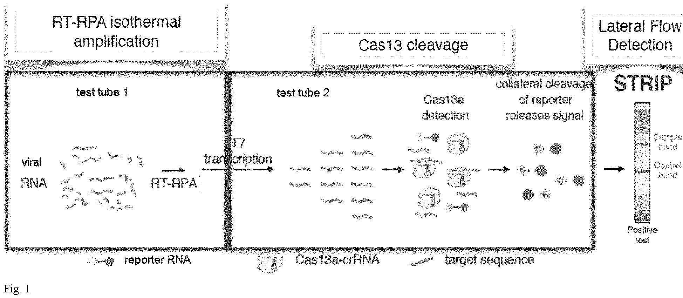

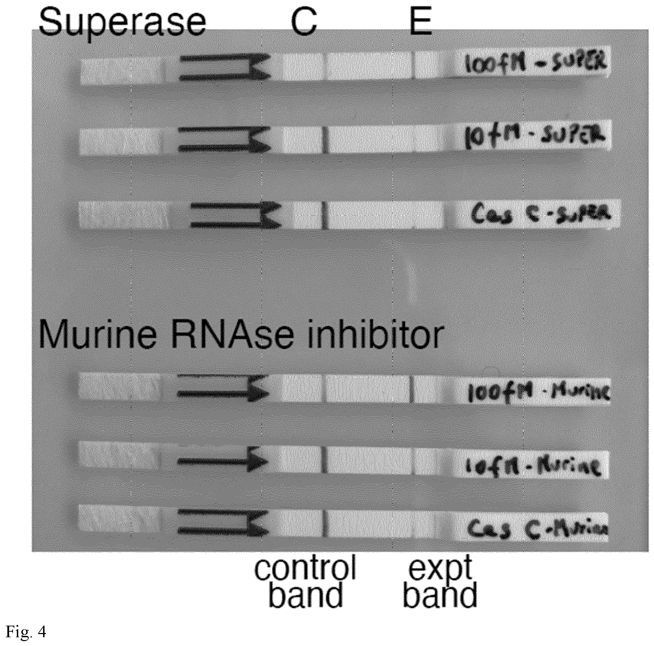

Release and amplification of viral RNA were performed in the same test tube (Fig. 1); SUPERase inhibitor (ThermoFisher Scientific cat# AM2694) was used as RNase inhibitor, and MuLV was found to be a better reverse transcriptase than ProtoScript (Fig. 4).

The sensitivity of the improved assay is at 5 copies of viral RNA/ µl if the Orf1a gene is targeted, and is at 0.5 copies of viral RNA/ µl if the S gene is targeted. This compares to 100 and 10 copies, respectively, for the method of Zhang et al.

When releasing is achieved by the non-ionic surfactant Oleth-8, the non-ionic surfactant can be used as a formulation commercialized by Lucigen (QuickExtract DNA Extraction Solution cat# QE09050) or NEB (Luna^{®} Cell Ready Lysis Module #E3032).

### Example 2: Optimization of reporter RNA amount

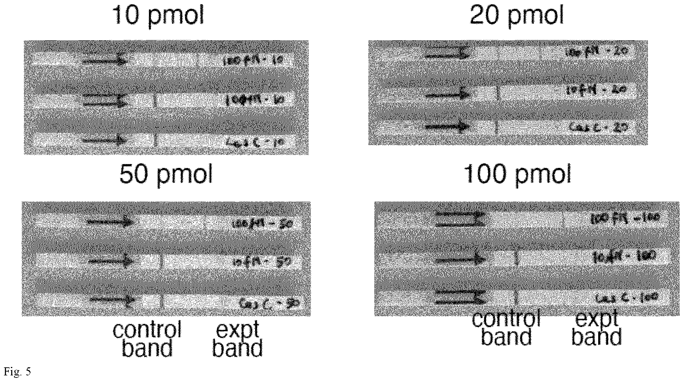

As shown in Fig. 5, the amount of reporter RNA per assay can be reduced to 10 pmol without loss of sensitivity. This compares to 200 pmol according to Zhang et al. (2020).

### Example 3: Comparison to gold standard in clinical samples

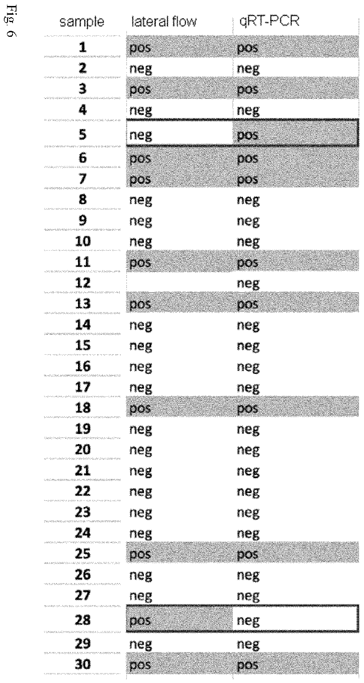

The improved assay was compared to results obtained by the present gold standard method (qRT-PCR) in clinical samples. The results were found to be identical, except for two samples close to the detection limit (Fig. 6).

### Example 3: Multiplex detection by fluorescence

In this example, the presence of SARS-CoV2 virus (S gene) and human RNAseP RNAs were detected at the same time. Cas13 and its crRNA, specific for S, detected the RNA produced by T7 polymerase from the amplicon produced during RPA amplification. Cas13 then was activated and cleaved an ssRNA reporter, with subsequent production of TEX615 fluorescent signal. Cas12 and its crRNA, specific for RNAseP, detected directly the dsDNA amplicon resulting from RPA amplification of RNasP mRNA. Upon recognition, Cas12 was activated and cleaved an ssDNA reporter, resulting in a FAM fluorescent signal. The Type V crRNA had the sequence 5'-rUrArA rUrUrU rCrUrA rCrUrA rArGrU rGrUrA rGrArU rGrArC rCrUrG rCrGrA rGrCrG rGrGrU rUrCrU rGrA-3' (SEQ ID NO:23), the reporter DNA had the sequence 5'-6-FAM-TTATTATTATT-Iowa Black^{®} FQ-3' (SEQ ID NO:19). The Type VI crRNA and the reporter RNA have been described herein above.

### Example 4: Multiplex detection by lateral flow

In this example, the presence of SARS-CoV2 virus (S gene) and human RNAseP RNAs are detected at the same time. Cas13 and its crRNA, specific for S, detect the RNA produced by T7 polymerase from the amplicon produced during RPA amplification. Cas13 then gets activated and cleaves a ssRNA reporter containing FAM and Biotin, respectively, at the 5'and 3' ends. Cas12 and its crRNA, specific for RNAseP, can detect directly the dsDNA amplicon resulting from RPA amplification of RNase P mRNA. Upon recognition, Cas12 get activated and cleaves an ssDNA reporter, containing FAM and Digoxigenin, respectively, at the 5'and 3' ends. The integrity of these reporters, and consequently, the activation state of Cas13 and Cas12, can be detected using HybriDetect 2T sticks(Milenia Biotec, MGHD2 1). The Type V crRNA was the same as in Example 3, the reporter DNA was 5'-6-FAM-TTATTATTATT-biotin-3' (SEQ ID NO:20) or 5'-6-FAM-TTATTATTATT-digoxigenin-3' (SEQ ID NO:21). The Type VI crRNA and the reporter RNA have been described herein above.

### Literature

- Chen et al. (2018), Science 360(6387):436
- Kellner et al. (2019), Nature Protocols 14:2986
- Wyllie et al. (2020), medRxiv 2020.04.16.20067835; doi.org/10.1101/2020.04.16.20067835
- Huang et al. (2020), Biosensors and Bioelectronics 164:112316
- Joung et al. (2020), "Point-of-care testing for COVID-19 using SHERLOCK diagnostics", v. 20200505, (www.stopcovid.science/docs/STOPCovid Whitepaper.pdf)
- Zhang et al. (2020), "SHERLOCK COVID-19 Testing Kit Instructions", v. 20200321, (www.broadinstitute.org/files/publications/special/COVID-19 detection (updated).pdf)
- Zhou et al. (2018), J Cell Mol Med 22:5807

## Claims

1. A method for detecting a target RNA and/or a target DNA in a sample of a subject, the method comprising the steps of
a) contacting said sample with a Type V Cas nuclease, a Type V crRNA targeting said target DNA, a type VI Cas nuclease, a Type VI crRNA targeting said target RNA, a reporter RNA and a reporter DNA; and
b) detecting cleavage of said reporter RNA, thereby detecting said target RNA; and/or detecting cleavage of said reporter DNA, thereby detecting said target DNA.

2. The method of claim 1, wherein said target RNA is at least a fragment of a genome of an RNA virus, of a pathogen RNA, or of an endogenous RNA of said subject, preferably an mRNA.

3. The method of claim 1 or 2, wherein said target DNA is (i) at least a fragment of a DNA comprised in said sample of said subject or (ii) a reverse transcript of an RNA comprised in said sample, preferably of an endogenous RNA, more preferably of an RNA of a housekeeping gene of said subject.

4. The method of any one of claims 1 to 3, wherein said target DNA is a control DNA for controlling extraction, preferably is a control DNA produced from said sample via reverse transcription and is for controlling extraction, reverse transcription and/or absence of RNases.

5. The method of any one of claims 1 to 4, wherein said target RNA is at least a fragment of a genome of an RNA virus and wherein said target DNA is a reverse transcript of an endogenous transcript of said subject, preferably of a transcript of a housekeeping gene of said subject.

6. The method of any one of claims 1 to 5, wherein said reporter RNA comprises a first and a second label, wherein the first label is attached towards the 5' end of the reporter RNA and the second label is attached towards the 3' end of the reporter RNA; and/or wherein said reporter DNA comprises a third and a fourth label, wherein the third label is attached towards the 5' end of the reporter DNA and the fourth label is attached towards the 3' end of the reporter DNA.

7. The method of any one of claims 1 to 6, wherein at least one of said first and second labels is an affinity label and/or wherein at least one of said third and fourth labels is an affinity label; or wherein at least one of said first and second labels is a quencher and/or wherein at least one of said third and fourth labels is a quencher.

8. The method of any one of claims 1 to 7, wherein detecting cleavage of said reporter RNA comprises detecting separation of the first label from the second label and/or wherein detecting cleavage of said reporter DNA comprises detecting separation of the second label from the fourth label.

9. The method of any one of claims 1 to 8, wherein said type VI Cas nuclease is a Cas13a nuclease, a Cas13b nuclease, a Cas13c nuclease, or a Cas13d nuclease, preferably is a Cas13a nuclease, more preferably a Cas13a nuclease from Leptotrichia wadeii (LwaCas13a) or from Leptotrichia buccalis (LbuCas13a) or from Capnocytophaga canimorsus Cc5 (CcaCas13b);

10. The method of any one of claims 1 to 9, wherein said type V Cas nuclease is a Cas12a nuclease, preferably from Lachnospiraceae bacterium ND2006 Cas12a (LbCas12a) or Acidaminococcus sp. BV3L6 (AsCas12a) or a Cas12b nuclease, preferably from Alicyclobacillus acidiphilus (AaCas12b).

11. A method for detecting an RNA virus in a sample, comprising the steps of
i)) releasing RNA from said sample;
ii) amplifying at least parts of the RNA comprised in said sample via a DNA intermediate;
iii) detecting a target RNA and a target DNA in the reaction mixture of step ii) according to the method according to any one of claims 1 to 10; and
iv) thereby detecting said RNA virus.

12. The method of claim 11, wherein said step ii) comprises amplifying said viral RNA by reverse-transcriptase recombinase polymerase amplification (RT-RPA).

13. The method of claim 11 or 12, wherein said RNA virus is selected from the list consisting of SARS-CoV-2, influenza virus, HCoV-NL63, HCoV-229E, HCoV-OC43, and HCoV-HKU1.

14. A type V Cas nuclease and/or a type VI Cas nuclease for use in diagnosing an infection with an RNA virus in a subject, preferably according to the method according to any one of claims 1 to 13.

15. A method for diagnosing an infection with an RNA virus in a subject, comprising
a) detecting an RNA virus in a sample of said subject by a method of any one of claims 1 to 13; and
b) in case an RNA virus is detected in step a), diagnosing an infection with an RNA virus in said subject.
